# EUROPEAN PATENT SPECIFICATION

(11) **EP 3 705 153 B1**
(45) Date of publication and mention of the grant of the patent: **23.10.2024**
(21) Application number: 19161019.5
(22) Date of filing: 06.03.2019
(51) Int. Cl.: A61M 25/00

(54) **INJECTION MOLDED CANNULA SYSTEM**
SPRITZGEGOSSENES KANÜLENSYSTEM
SYSTÈME DE CANULE MOULÉ PAR INJECTION

(43) Date of publication of application: 09.09.2020
(73) Proprietor: Roche Diabetes Care GmbH, 68305 Mannheim (DE); F. Hoffmann-La Roche AG, 4070 Basel (CH)
(72) Inventor: FREITAG, Christian, 68305 Mannheim (DE); RASMUSSEN, Mads, 68305 Mannheim (DE)
(74) Representative: Rentsch Partner AG

(56) References cited:
- WO-A2-99/21605
- DE-C1- 3 530 349
- US-A- 4 430 081
- US-A- 4 781 703

## Description

### Field of disclosure

The present invention lies in the filed on infusion technology. More particularly, it is related to infusion site interfaces and infusion pumps, as well as manufacturing methods for cannula systems for infusion site interfaces.

### Background, prior art

infusion pumps are used for parenterally providing patients with liquid medicaments over longer time periods. Nowadays, infusion pumps with very small dimensions are available that can be carried by the patient on the body. Such small-sized ambulatory infusion pumps are particularly useful for metering small doses of highly effective liquid medicaments, such as insulin for the treatment of diabetes, or analgesics for pain therapy, which are conveyed through a cannula into the tissue of a patient.

In one approach, an infusion pump, carried somewhere on the body, e.g. attached to a belt, is fluidly connected via flexible tubing to an infusion site interface, also called insertion head, that is attached to the body of the patient. The infusion site interface comprises a cannula system with a cannula to be inserted into the body tissue, a housing and connector means for fluidly connecting the cannula with the flexible tubing connected to the upstream infusion pump. The tubing can be repeatedly connected and disconnected from the infusion site interface. The connector means may for example comprise a septum sealingly closing the fluid system of cannula and housing. The septum can be penetrated by a hollow needle, for reversibly establishing a fluid connection. The cannula is preferably made of a flexible material and thus a soft cannula. Such cannulas are more comfortable for their users, particularly during body movements. Since flexible cannulas cannot be inserted directly into the tissue, an additional piercing device, e.g. in the form of a rigid piercing needle made from metal, is arranged inside the flexible cannula. A pointed end of the piercing device protrudes from the proximal end of the cannula, the cannula that will be open toward the interstitial fluid. After inserting the piercing device and the stabilized cannula into the body tissue, the rigid piercing device is removed from the flexible cannula. The cannula remains in the body tissue. Generally, a piercing needle is arranged in such a way that it penetrates a septum, which after withdrawal of the piercing needle sealingly closes the distal end of the now open cannula fluid path.

In another approach, the infusion pump device is directly fluidly connected with the infusion site interface. The fluid connection between pump and cannula is established by a hollow connector needle of the pump, reversibly penetrating a septum of the cannula unit that sealingly closes the distal end of the cannula fluid path. Advantageously, the pump can be repeatedly connected and disconnected from the infusion site interface.

In a common method for manufacturing infusion site interfaces with soft and flexible cannulas, a stabilizing pin is introduced in a first step into the flexible cannula, for simplifying the handling of the flexible cannula during the manufacturing process. The temporary structural unit prepared in this manner is inserted into a previously manufactured housing, the flexible cannula and the housing are permanently connected, for example by a thermal process. The stabilizing pin is then drawn out of the flexible cannula, and is replaced by the actual piercing device. The insertion of the piercing devices sometimes damages the flexible cannulas, which results in comparably high rejection rates during manufacturing.

WO 2018 033 614 A1 discloses a cannula unit comprising a housing and a flexible cannula. The flexible cannula is provided with an end area that deviates from the shape of the rest of the flexible cannula. The end area of the flexible cannula has for example a funnel-like shape or the shape of the flange. Furthermore, the housing comprises two parts, between which the end area of the flexible cannula is positively locked to the housing by clamping. The two parts of the housing are ultimately connected by welding, particularly laser welding.

Further prior art is known from WO 99/21605 A2, US 4 781 703 A, US 4 430 081 A and DE 35 30 349 C1.

### Summary of disclosure

Cannula units and systems known in the state of the art suffer from certain drawbacks. For example, if the cannula unit comprises a housing with two parts, which are separately produced and only subsequently connected during the manufacturing process, certain restrictions are imposed. Firstly, the materials have to be suitable for welding or gluing, such that the two housing parts can be connected. Secondly, the connecting step represents an additional step, which increases the manufacturing time and cost of the production. Thirdly, the two parts of the housing must be strictly complementary to each other, thus allowing only minor material tolerances, as otherwise leakage becomes a problem. This is a severe problem in the state of the art, as the amount of deficient products due to non-complementary housing parts is significantly high.

It is therefore an overall objective of the present invention to improve the state of the art regarding the design and use of cannula systems in the context of infusion of liquid drugs, thereby preferably avoiding disadvantages of the prior art fully or partly.

In advantageous embodiments, a cannula unit is provided, in which the occurrence of leakage is avoided or at least significantly reduced.

In further favorable embodiments, a cannula unit is provided, which can be manufactured in a time and cost efficient manner.

In additional favorable embodiments, the amount of deficient products during the manufacturing process and thus the rejection rate is reduced.

According to a first object of the invention, the overall objective is achieved by a method for manufacturing a cannula system with a body unit and a compressing unit. The method comprises providing the body unit comprising a mounting structure, the body unit being mounted onto a molding core pin. Subsequently, a soft cannula is threaded on the mounting structure of the body unit. In a following step, the compressing unit is injection molded on the soft cannula and at least on parts of the body unit. The compressing unit circumferentially surrounds the mounting structure, at least parts of the soft cannula and at least parts of the body unit. Furthermore, the injection-molded compressing unit is cooled, thereby providing an internal material tension of the compressing unit. The cooling typically entails shrinking of the compressing unit. In addition, the molding core pin is removed. It is understood by the skilled person that the molding core pin can be removed either directly after injection molding of the compressing unit, i.e. before cooling of the compressing unit, after cooling the compressing unit or even during cooling of the compressing unit.

The body unit is preferably also produced by injection molding. For example, the body may be provided by injection molding onto the molding core pin. However, the body unit may also be produced by another suitable method. Particularly, if the body unit is produced by injection molding, the two injection molding steps may be conducted as two separate injection molding steps.

Using injection-molding technology for manufacturing the compressing unit and optionally the body unit has the advantage that only a reduced number of tools is required. Furthermore, no additional connection step such as welding or gluing, is required. In addition, as the compressing unit is injection molded on the body unit and the cannula and subsequently cooled, shrinking induces an internal material tension, which significantly increases the fixture of the soft cannula within the cannula system. The shrinking and the thus associated internal material tension can be increased, if cooling is performed in the mold. Furthermore, injection molding of both the body unit and the compressing unit has the advantage that the two parts are necessarily complementary to each other and thus the amount of material deficient parts is significantly reduced.

It is understood that the term "circumferentially" does not necessarily require that the mounting structure, the cannula and or the body unit have a round cross section and/or a cylindrical shape. These components may independently of each other comprise a round, elliptical or angular cross section and further have any shape suitable for an infusion set.

In some embodiments the compressing unit is injection molded such that it circumferentially surrounds at least 1/3, preferably 1/2 of the outer surface of the body unit, thereby providing a particular tight and safe connection between the body unit and the compressing unit.

In further embodiments, only a distal end area of the soft cannula is threaded on the mounting structure of the body unit. The compressing unit preferably circumferentially surrounds only the distal end area of the soft cannula. As the skilled person understands, the distal end area is facing away from the patient in an operative state. Thus, the distal end area is closer to the body unit as the proximal end area. Upon insertion into the patient's tissue, the proximal end area is therefore first inserted into the tissue.

In other embodiments, the distal end area of the soft cannula has a larger diameter than the rest of the cannula before the cannula is threaded on the mounting structure. Thus, the cannula is, when pushed over the mounting structure, not or only slightly laterally expanded, which will result in no or less material creep and potentially leads to less leakage.

In further embodiments, the internal material tension of the compressing unit exerts a compression force which is directed radially inwards. Such a force is particularly advantageous, as the risk of leakage between the cannula and the mounting structure and/or between the cannula and the compressing unit is further decreased.

In other embodiments, the internal material tension of the compressing unit compresses the parts of the soft cannula threaded on the mounting structure. In such embodiments, at least the part of the cannula which is circumferentially surrounded by the compressing unit, preferably the distal end area, is compressed. Thus, the wall thickness of the cannula of this part is smaller than the wall thickness of the rest of the cannula which is not circumferentially surrounded by the compressing unit.

In further embodiments, the body unit and the compressing unit and/or the compressing unit and the soft cannula form a bonded connection during injection molding of the compressing unit.

In other embodiments, the methods consists of two separate injection molding steps, thereby reducing the required amount of manufacturing steps and the overall production time and costs even further.

According to a second aspect of the invention, the overall objective is achieved by a cannula system comprising a soft cannula and a cannula unit for holding the soft cannula. The cannula unit comprises or alternatively may also consist of a compressing unit and a body unit, wherein the body unit contains a mounting structure and a cavity for holding a septum. The soft cannula is threaded on the mounting structure of the body unit. Furthermore, the compressing unit circumferentially surrounds the mounting structure and at least parts of the body unit. The compressing unit also comprises an internal material tension for exerting a compression force on the soft cannula and the mounting structure.

For example, such a cannula system may be manufactured by a method as described herein. Furthermore, the body unit and/or the compressing unit are typically injection molded.

In some embodiments, the mounting structure may be a protuberance, in particular a cylindrical or cubical protuberance.

In further embodiments, only a distal end area of the soft cannula is threaded on the mounting structure and the compressing unit circumferentially surrounds the distal end area of the soft cannula.

In other embodiments, the distal end area of the soft cannula has a larger diameter than the rest of the soft cannula. Thus, in these embodiments, the whole distal area has a larger diameter than the rest of the soft cannula.

Typically, the distal end area of the soft cannula is free of an internal material tension.

In some embodiments, the wall thickness of the distal end area of the soft cannula is smaller than the wall thickness of the rest of the soft cannula.

In other embodiments, the compressing unit compresses at least the distal end area of the soft cannula. In these embodiments, a particular fluid tight system is achieved and the risk of leakage is reduced.

In further embodiments, the body unit and the compressing unit are connected by a bonded connection and/or the soft cannula and the compressing unit are connected by a bonded connection.

It is understood that such a bonded connection is typically only formed by the body unit, the compressing unit and/or the soft cannula itself. Thus an external adhesive, for example a glue, is neither required nor present.

In some embodiments, the cannula system does not comprise additional connection means, particularly form-lock means, for coupling and/or connecting the compressing unit and the body unit such as for example barbs, nipples, snap fits and the like.

In further embodiments, the body unit may comprise a septum within the cavity for holding the septum. The septum may be fixed by crimping. Such a septum is typically free of a slit prior to insertion of a piercing needle. In particular, such a septum may not comprise a slit elongated in both the lateral and the vertical piercing direction of the septum.

According to another aspect of the invention, the overall objective is achieved by the use of a cannula system according to any of the embodiments described herein in an infusion set.

### Brief description of the figures

Figure 1 shows a cross-sectional view of a cannula system in accordance to an embodiment of the invention.
Figures 2a to 2c schematically show the manufacturing method of a cannula system according to an embodiment of the invention.

### Exemplary embodiments

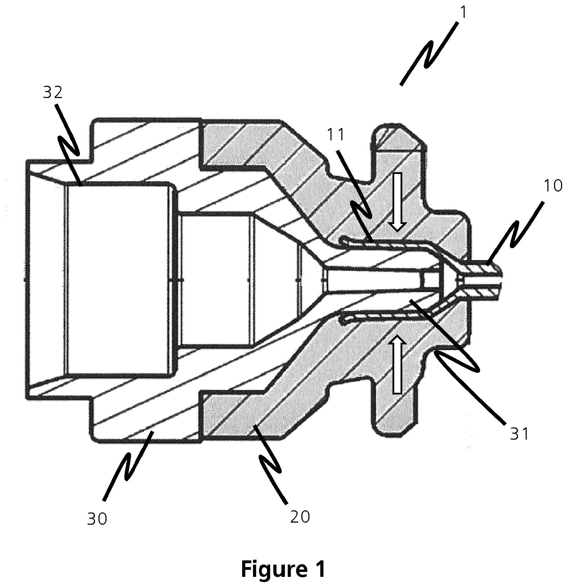

Figure 1 shows an advantageous embodiment of a cannula system 1 according to the invention. Cannula system 1 comprises soft cannula 10 and a cannula unit for holding soft cannula 10. The cannula unit comprises compressing unit 20 and body unit 30. Body unit 30 further comprises mounting structure 31 and cavity 32 for receiving a septum. Distal part 11 of soft cannula 10 is threaded on mounting structure 31. The diameter of distal end area 11 of soft cannula 10 is larger than the diameter of the rest of the soft cannula. Compressing unit 20 circumferentially surrounds distal end area 11 of soft cannula 10, mounting structure 31 and also an additional part of body unit 30. As shown in Figure 1, more than 1/3 of the surface of the body unit are circumferentially surrounded by compressing unit 20.

As indicated by the arrows, the compressing unit 20 has an internal material tension, thereby exerting a radially inwards directed compression force on the distal end area of the soft cannula and the mounting structure. As a result, a particular tight connection between soft cannula 10, mounting structure 31 and compressing unit 20 is achieved. Furthermore, as can be readily seen in Figure 1, the wall thickness of distal end area 11 of soft cannula 10 is smaller than the wall thickness of the rest of soft cannula 10. In addition, as indicated by the arrows, distal end area 11 of soft cannula 10 is compressed by the compression force exerted by compressing unit 20. Compressing unit 20 and body unit 30 are connected by a bonding connection at their contact area. As a result, a reliable and sealingly tight connection is established.

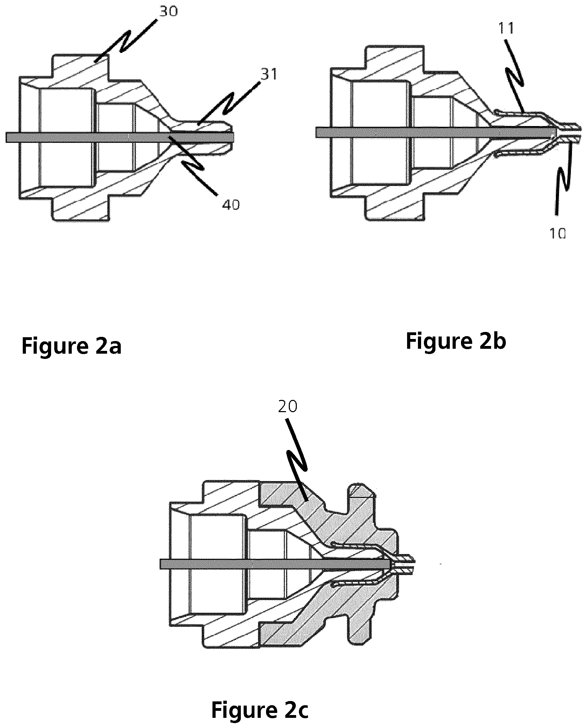

Figures 2a to 2c depict certain steps of a method of manufacturing a cannula system according to an advantageous embodiment of the invention. As shown in Figure 2a, body unit 30 with mounting structure 31 is injection molded onto a molding core pin 40 (mold not shown). In a following step, distal end area 11 of soft cannula 10 is threaded on mounting structure 31 (Figure 2b). Afterwards, compressing unit 20 is injection molded on the distal end area of the soft cannula, on the mounting structure and also on further parts of body unit 30 (Figure 2c, mold not shown). As the compressing unit is at least partially injection molded on body unit 30, any production imprecision of the proximal face of the body unit is less relevant, as the compressing unit is necessarily complementary to those parts of the body unit 30. Once compressing unit has been injection molded, molding core pin 40 can be removed and the compressing unit be cooled, upon which an internal material tension is established, thereby exerting a force on distal end area 11 of soft cannula 10 and mounting structure 31. Generally, the cooling can be performed in the mold and/or the cooling may be achieved by active cooling. Thus, the compressing unit is not allowed to cool to room temperature by itself, but actively cooled, particularly by using a cooling medium. Such rapid cooling enhances the internal material tension. The two-step injection molding sequence can be performed significantly faster as the methods for manufacturing cannula unit as known in the state of the art.

## Claims

1. Method for manufacturing a cannula system (1) with a body unit (30) and a compressing unit (20), the method comprising the steps:
- Providing a body unit (30) comprising a mounting structure (31), the body unit (30) being mounted onto a molding core pin (40);
- Threading a soft cannula (10) on the mounting structure (31) of the body unit (30);
- injection molding of the compressing unit (20) on the soft cannula (10) and at least parts of the body unit (30), wherein the compressing unit (20) circumferentially surrounds the mounting structure (31), at least parts of the soft cannula (10) and at least parts of the body unit (30);
- Cooling of the compressing unit (20), thereby providing an internal material tension of the compressing unit (20);
- Removing the molding core pin (40).

2. The method according to claim 1, wherein the body unit (30) is provided by injection molding the body unit (30) onto the molding core pin (40).

3. The method according to claim 1 or 2, wherein only a distal end area (11) of the soft cannula (10) is threaded on the mounting structure (31) of the body unit (30) and wherein the compressing unit (20) preferably circumferentially surrounds only the distal end area (11) of the soft cannula (10).

4. The method according to claim 3, wherein the distal end area (11) of the soft cannula (10) has a larger diameter than the rest of the cannula before the cannula (10) is threaded on the mounting structure (31).

5. The method according to any of the previous claims, wherein the internal material tension of the compressing unit (20) exerts a compression force which is directed radially inwards.

6. The method according to any of the previous claims, wherein the internal material tension of the compressing unit (20) compresses the soft cannula.

7. The method according to any of the previous claims, wherein the body unit (30) and the compressing unit (20) and/or wherein the compressing unit (20) and the soft cannula (10) form a bonded connection during injection molding of the compressing unit (20).

8. The method according to any of the previous claims, wherein the method consists of two separate injection molding steps.

9. Cannula system (1) comprising a soft cannula (10) and a cannula unit for holding the soft cannula (10), wherein the cannula unit comprises a compressing unit (20) and a body unit (30), wherein the body unit (30) contains a mounting structure (31) and a cavity (32) for holding a septum, wherein the soft cannula (10) is threaded on the mounting structure (31), wherein the compressing unit (20) circumferentially surrounds the mounting structure (31) and at least parts of the body unit (30) and wherein the compressing unit (20) is injection molded on the soft cannula (10) and at least parts of the body unit (30) and wherein the compressing unit (20) contains an internal material tension for exerting a compression force on the soft cannula (10) and the mounting structure (31).

10. Cannula system (1) according to claim 9, wherein only a distal end area (11) of the soft cannula (10) is threaded on the mounting structure (31) and wherein the compressing unit (20) circumferentially surrounds the distal end area (11) of the soft cannula (10).

11. Cannula system (1) according to claim 10, wherein distal end area (11) of the soft cannula (10) has a larger diameter than the rest of the soft cannula (10).

12. Cannula system (1) according to any of claims 10 or 11, wherein the compressing unit (20) compresses at least the distal end area (11) of the soft cannula (10).

13. Cannula system (1) according to any of claims 9 to 12, wherein the body unit (30) and the compressing unit (20) are connected by a bonded connection and/or wherein the soft cannula (10) and the compressing unit (20) are connected by a bonded connection.

14. Use of a cannula system according to any of claims 9 to 13 in an infusion set.

## Patentansprüche

1. Verfahren zur Herstellung eines Kanülensystems (1) mit einer Körpereinheit (30) und einer Kompressionseinheit (20), wobei das Verfahren die folgenden Schritte umfasst:
- Bereitstellen einer Körpereinheit (30), umfassend eine Befestigungsstruktur (31), wobei die Körpereinheit (30) auf einem Formkernstift (40) befestigt ist;
- Schrauben einer Weichkanüle (10) auf die Befestigungsstruktur (31) der Körpereinheit (30);
- Spritzgießen der Kompressionseinheit (20) auf die Weichkanüle (10) und mindestens Teile der Körpereinheit (30), wobei die Kompressionseinheit (20) die Befestigungsstruktur (31), mindestens Teile der Weichkanüle (10) und mindestens Teile der Körpereinheit (30) umfänglich umschließt;
- Abkühlen der Kompressionseinheit (20), wodurch eine innere Materialspannung der Kompressionseinheit (20) bereitgestellt wird;
- Entfernen des Formkernstifts (40).

2. Verfahren nach Anspruch 1, wobei die Körpereinheit (30) durch Spritzgießen der Körpereinheit (30) auf den Formkernstift (40) bereitgestellt wird.

3. Verfahren nach Anspruch 1 oder 2, wobei nur ein distaler Endbereich (11) der Weichkanüle (10) auf die Befestigungsstruktur (31) der Körpereinheit (30) geschraubt wird und wobei die Kompressionseinheit (20) vorzugsweise nur den distalen Endbereich (11) der Weichkanüle (10) umfänglich umschließt.

4. Verfahren nach Anspruch 3, wobei der distale Endbereich (11) der Weichkanüle (10) einen größeren Durchmesser hat als der Rest der Kanüle, bevor die Kanüle (10) auf die Befestigungsstruktur (31) geschraubt wird.

5. Verfahren nach einem der vorstehenden Ansprüche, wobei die innere Materialspannung der Kompressionseinheit (20) eine Kompressionskraft ausübt, die radial nach innen gerichtet ist.

6. Verfahren nach einem der vorstehenden Ansprüche, wobei die innere Materialspannung der Kompressionseinheit (20) die Weichkanüle komprimiert.

7. Verfahren nach einem der vorstehenden Ansprüche, wobei die Körpereinheit (30) und die Kompressionseinheit (20) und/oder wobei die Kompressionseinheit (20) und die Weichkanüle (10) eine Haftverbindung während des Spritzgießens der Kompressionseinheit (20) bilden.

8. Verfahren nach einem der vorstehenden Ansprüche, wobei das Verfahren aus zwei separaten Spritzgießschritten besteht.

9. Kanülensystem (1), umfassend eine Weichkanüle (10) und eine Kanüleneinheit zum Aufnehmen der Weichkanüle (10), wobei die Kanüleneinheit eine Kompressionseinheit (20) und eine Körpereinheit (30) umfasst, wobei die Körpereinheit (30) eine Befestigungsstruktur (31) und einen Hohlraum (32) zum Aufnehmen eines Septums enthält, wobei die Weichkanüle (10) auf die Befestigungsstruktur (31) geschraubt ist, wobei die Kompressionseinheit (20) die Befestigungsstruktur (31) und mindestens Teile der Körpereinheit (30) umfänglich umschließt und wobei die Kompressionseinheit (20) auf die Weichkanüle (10) und mindestens Teile der Körpereinheit (30) spritzgegossen ist und wobei die Kompressionseinheit (20) eine innere Materialspannung zum Ausüben einer Kompressionskraft auf die Weichkanüle (10) und die Befestigungsstruktur (31) aufweist.

10. Kanülensystem (1) nach Anspruch 9, wobei nur ein distaler Endbereich (11) der Weichkanüle (10) auf die Befestigungsstruktur (31) geschraubt ist und wobei die Kompressionseinheit (20) den distalen Endbereich (11) der Weichkanüle (10) umfänglich umschließt.

11. Kanülensystem (1) nach Anspruch 10, wobei der distale Endbereich (11) der Weichkanüle (10) einen größeren Durchmesser hat als der Rest der Weichkanüle (10).

12. Kanülensystem (1) nach einem der Ansprüche 10 oder 11, wobei die Kompressionseinheit (20) mindestens den distalen Endbereich (11) der Weichkanüle (10) komprimiert.

13. Kanülensystem (1) nach einem der Ansprüche 9 bis 12, wobei die Körpereinheit (30) und die Kompressionseinheit (20) über eine Haftverbindung verbunden sind und/oder wobei die Weichkanüle (10) und die Kompressionseinheit (20) über eine Haftverbindung verbunden sind.

14. Verwendung eines Kanülensystems nach einem der Ansprüche 9 bis 13 in einem Infusionsbesteck.

## Revendications

1. Procédé de fabrication d'un système de canule (1) avec une unité de corps (30) et une unité de compression (20), le procédé comprenant les étapes :
- fourniture d'une unité de corps (30) comprenant une structure de montage (31), l'unité de corps (30) étant montée sur une broche-noyau de moulage (40) ;
- filetage d'une canule souple (10) sur la structure de montage (31) de l'unité de corps (30) ;
- moulage par injection de l'unité de compression (20) sur la canule souple (10) et au moins des parties de l'unité de corps (30), l'unité de compression (20) entourant de manière circonférentielle la structure de montage (31), au moins des parties de la canule souple (10) et au moins des parties de l'unité de corps (30) ;
- refroidissement de l'unité de compression (20), fournissant ainsi une tension matérielle interne de l'unité de compression (20) ;
- retrait de la broche-noyau de moulage (40).

2. Procédé selon la revendication 1, dans lequel l'unité de corps (30) est fournie par moulage par injection de l'unité de corps (30) sur la broche-noyau de moulage (40).

3. Procédé selon la revendication 1 ou 2, dans lequel uniquement une zone d'extrémité distale (11) de la canule souple (10) est filetée sur la structure de montage (31) de l'unité de corps (30) et dans lequel l'unité de compression (20) entoure de préférence de manière circonférentielle uniquement la zone d'extrémité distale (11) de la canule souple (10).

4. Procédé selon la revendication 3, dans lequel la zone d'extrémité distale (11) de la canule souple (10) a un plus grand diamètre que le reste de la canule avant le filetage de la canule (10) sur la structure de montage (31).

5. Procédé selon l'une quelconque des revendications précédentes, dans lequel la tension matérielle interne de l'unité de compression (20) exerce une force de compression qui est dirigée radialement vers l'intérieur.

6. Procédé selon l'une quelconque des revendications précédentes, dans lequel la tension matérielle interne de l'unité de compression (20) comprime la canule souple.

7. Procédé selon l'une quelconque des revendications précédentes, dans lequel l'unité de corps (30) et l'unité de compression (20) et/ou dans lequel l'unité de compression (20) et la canule souple (10) forment un assemblage lié pendant le moulage par injection de l'unité de compression (20).

8. Procédé selon l'une quelconque des revendications précédentes, dans lequel le procédé est constitué de deux étapes de moulage par injection séparées.

9. Système de canule (1) comprenant une canule souple (10) et une unité de canule pour retenir la canule souple (10), dans lequel l'unité de canule comprend une unité de compression (20) et une unité de corps (30), dans lequel l'unité de corps (30) contient une structure de montage (31) et une cavité (32) pour retenir un septum, dans lequel la canule souple (10) est filetée sur la structure de montage (31), dans lequel l'unité de compression (20) entoure de manière circonférentielle la structure de montage (31) et au moins des parties de l'unité de corps (30) et dans lequel l'unité de compression (20) est moulée par injection sur la canule souple (10) et au moins des parties de l'unité de corps (30) et dans lequel l'unité de compression (20) contient une tension matérielle interne pour exercer une force de compression sur la canule souple (10) et la structure de montage (31).

10. Système de canule (1) selon la revendication 9, dans lequel uniquement une zone d'extrémité distale (11) de la canule souple (10) est filetée sur la structure de montage (31) et dans lequel l'unité de compression (20) entoure de manière circonférentielle la zone d'extrémité distale (11) de la canule souple (10).

11. Système de canule (1) selon la revendication 10, dans lequel la zone d'extrémité distale (11) de la canule souple (10) a un plus grand diamètre que le reste de la canule souple (10).

12. Système de canule (1) selon l'une quelconque des revendications 10 ou 11, dans lequel l'unité de compression (20) comprime au moins la zone d'extrémité distale (11) de la canule souple (10).

13. Système de canule (1) selon l'une quelconque des revendications 9 à 12, dans lequel l'unité de corps (30) et l'unité de compression (20) sont assemblées par un assemblage lié et/ou dans lequel la canule souple (10) et l'unité de compression (20) sont assemblées par un assemblage lié.

14. Utilisation d'un système de canule selon l'une quelconque des revendications 9 à 13 dans un ensemble de perfusion.
